# EUROPEAN PATENT SPECIFICATION

(11) **EP 3 835 259 B1**
(45) Date of publication and mention of the grant of the patent: **18.09.2024**
(21) Application number: 19847034.6
(22) Date of filing: 02.08.2019
(51) Int. Cl.: C01B 21/064, A61K 8/02, A61K 8/19, A61Q 1/02, C08K 3/38

(54) **HEXAGONAL BORON NITRIDE POWDER**
HEXAGONALES BORNITRIDPULVER
POUDRE DE NITRURE DE BORE HEXAGONAL

(30) Priority: 07.08.2018 JP 2018148821
(43) Date of publication of application: 16.06.2021
(73) Proprietor: JFE Mineral & Alloy Company, Ltd., Tokyo 105-0014 (JP)
(72) Inventor: NAKAGAWA Yumi, Kurashiki-shi, Okayama 712-8513 (JP); KURODA Masaomi, Kurashiki-shi, Okayama 712-8513 (JP); KATAYAMA Shigeyuki, Kurashiki-shi, Okayama 712-8513 (JP); NABESHIMA Seiji, Kurashiki-shi, Okayama 712-8513 (JP); MIYAGUCHI Masashi, Kurashiki-shi, Okayama 712-8513 (JP)
(74) Representative: HGF
(86) International application number: PCT/JP2019/030572
(87) International publication number: WO 2020/031913

(56) References cited:
- WO-A2-2012/027194
- JP-A- 2012 176 910
- JP-A- 2015 140 337
- JP-A- 2017 160 086
- JP-A- H05 186 205
- DATABASE WPI Week 201711, Derwent World Patents Index; AN 2017-06217X, XP002804216
- DATABASE WPI Week 201705, Derwent World Patents Index; AN 2016-81481U, XP002804222
- DATABASE WPI Week 201263, Derwent World Patents Index; AN 2012-L87422, XP002804223
- DATABASE WPI Week 199334, Derwent World Patents Index; AN 1993-269653, XP002804224

## Description

### TECHNICAL FIELD

This disclosure relates to a hexagonal boron nitride powder, in particular, to a hexagonal boron nitride powder that has excellent glitter property and that is suitable for use in cosmetics.

### BACKGROUND

Hexagonal boron nitride (h-BN) is a compound having a graphite-like layered structure in which hexagonal network layers composed of boron (B) and nitrogen (N) are stacked on top of one another. Accordingly, particles of general hexagonal boron nitride have a flat plate-like (scaly) structure. In addition, no covalent bond is present across the layers in the particles, and the force acting across the layers is only the van der Waals force, which is an extremely weak force. Therefore, slippage occurs between layers with a slight force, and consequently hexagonal boron nitride powder has extremely excellent lubricity.

In addition, since hexagonal boron nitride is chemically stable and does not adversely affect the human body, it is widely used as one of body pigments for cosmetics excellent in "spreadability" when applied to a skin surface by taking advantage of the above-mentioned lubricity (WO2013/065556A (PTL 1), and WO2014/049956A (PTL 2)).
JP2017014064A (PTL 5) discloses hexagonal boron nitride particles, hexagonal boron nitride powder containing the particles, and a method for producing the same. Specifically, the crystal having a hexagonal crystal morphology at two or more bent portions contains hexagonal boron nitride particles having a continuous polyhedral structure, and imparts high thermal conductivity to the resin composition obtained by filling the resin.
JP2016216271A (PTL 6) discloses hexagonal boron nitride particles and hexagonal boron nitride powder containing the same. Specifically, it contains novel hexagonal boron nitride particles having a twin structure and the hexagonal boron nitride particles, and when filled in a resin, the thermal anisotropy derived from the plate-like crystals of hexagonal boron nitride is reduced. Furthermore, PTL 6 provides a hexagonal boron nitride powder capable of exhibiting high insulation resistance by suppressing entrainment of bubbles seen in agglomerated particles.

### CITATION LIST

### Patent Literature

PTL 1: WO2013/065556A
PTL 2: WO2014/049956A
PTL 3: JP2001-011340A
PTL 4: JP2010-163371A
PTL 5: JP2017014064A
PTL 6: JP2016216271A

### SUMMARY

### (Technical Problem)

Depending on the application, cosmetics may be required to have excellent glitter property in addition to lubricity. In such cases, for example, as described in JP2001-011340A (PTL 3) and JP2010-163371A (PTL 4), it has been proposed to impart glitter property by adding a glitter pigment such as glass flakes or a lamellar agent to the cosmetics.

However, when using a conventional hexagonal boron nitride powder in combination with a glitter pigment, although the effect of giving a smooth feel and shine to the cosmetics is retained, there is a problem that the inherent glitter property of the glitter pigment is not fully obtained.

In view of the above situations, it would thus be helpful to provide a hexagonal boron nitride powder that offers both a smooth feel, which is a characteristic of boron nitride powder, and excellent glitter property, and that is capable of retaining excellent glitter property when used in combination with a glitter pigment.

### (Solution to Problem)

The present inventors focused on the shape of hexagonal boron nitride powder, based on the idea that a decrease in glitter experienced with the use of a boron nitride powder in combination with a glitter pigment is ascribable to the interference between the light reflected by the glitter pigment and the light reflected by the hexagonal boron nitride powder. Then, as a result of further investigations to address the above issues, the following findings (1) and (2) were obtained.
(1) By increasing the proportion of boron nitride particles in a boron nitride powder that have a bent structure at a specific angle, it is possible to improve not only the glitter property of the boron nitride powder when used alone, but also the glitter property when used in combination with a glitter pigment.
(2) By adding one or both of carbonates of alkali metals and carbonates of alkaline earth metals to the raw material used to produce a boron nitride powder and by preparing a hexagonal boron nitride powder under certain conditions, it is possible to produce a hexagonal boron nitride powder satisfying the above condition (1).

The present disclosure was completed based on these findings, and primary features thereof are as follows.
1. A hexagonal boron nitride powder comprising hexagonal boron nitride particles, wherein among the hexagonal boron nitride particles, a number ratio of particles having a bent structure at an angle of 110° to 160° with respect to (0,0,1) crystal plane of the particles is 30 % or more; the proportion of particles having a particle size of 200 µm or more is 0.5 mass% or less; and the avearge apparent thickness of the hexagonal boron nitride particles is 0.5 µm to 3.0 µm.
2. The hexagonal boron nitride powder according to 1., wherein among the hexagonal boron nitride particles, a number ratio of particles having a bent structure at an angle of 110° to 130° with respect to (0,0,1) crystal plane of the particles is 60 % or more.
3. The hexagonal boron nitride powder according to 1. or 2., wherein a full width at half maximum of a reflectance peak measured with a variable angle photometer at an angle of incident light of 45° is 80° or less.
4. The hexagonal boron nitride powder according to any one of 1. to 3., having an mean particle size of 6 µm to 100 µm.
5. The hexagonal boron nitride powder according to any one of 1. to 4., being suitable for cosmetic use.

### (Advantageous Effect)

The hexagonal boron nitride powder disclosed herein has excellent glitter property, in addition to the lubricity inherent to hexagonal boron nitride powder, which makes it extremely suitable for cosmetic use.

### BRIEF DESCRIPTION OF THE DRAWINGS

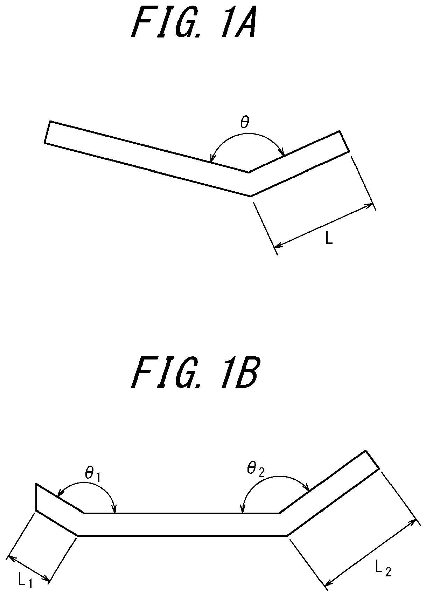

FIGS. 1A and 1B schematically illustrate exemplary particle shapes according to an embodiment of the present disclosure.

### DETAILED DESCRIPTION

### [Bent Particle Ratio]

It is important in the present disclosure to set a number ratio of particles having a bent structure at an angle of 110° to 160° with respect to (0,0,1) crystal plane of the particles among the hexagonal boron nitride particles contained in the hexagonal boron nitride powder (hereinafter referred to as a "bent particle ratio") to 30 % or more. If the bent particle ratio is less than 30 %, sufficient glitter property cannot be obtained. Therefore, the bent particle ratio is set to 50 % or more, preferably 70 % or more, and more preferably 80 % or more. On the other hand, the upper limit of the bent particle ratio is not particularly limited, and may be 100 %. However, since this effect is saturated when the bent particle ratio exceeds 90 %, the bent particle ratio may be 90 % or less. The bent particle ratio can be measured according to the method described in the EXAMPLES section below. The hexagonal boron nitride powder satisfying the above conditions can be produced according to the production method described later.

A number ratio of particles having a bent structure at an angle of 110° to 130° with respect to (0,0,1) crystal plane of the particles among the hexagonal boron nitride particles contained in the hexagonal boron nitride powder (hereinafter referred to as a "second bent particle ratio") is preferably 60 % or more. The second bent particle ratio can be increased, for example, by increasing the amount of additive(s) in the production method described later.

### [Length of Bent Portion]

The length of a bent portion of each particle having a bent structure at an angle of 110° to 160° with respect to (0,0,1) crystal plane of the particles is not particularly limited, yet is preferably 3 µm or more. This is because the bending effect cannot be obtained if the length is less than 3 µm. As used herein, the "length of a bent portion" refers to an average distance from an apex of a bend to one of end faces closer to the apex on an outer surface of the bend when a particle is observed under a microscopic field of view.

For example, as illustrated in FIG. 1A, in the case of a particle having a single bend at an angle of 110° to 160°, the bent portion preferably has a length L of 3 µm or more. In this case, an average distance from an apex of the bend to one of end faces farther from the apex is also preferably 3 µm or more.

In addition, as illustrated in FIG. 1B, in the case of a particle having two or more bends at an angle of 110° to 160°, it is preferable that at least one of a length L₁ of a bent portion of one of the bends closest to one end face or a length L₂ of a bent portion of one of the bends closest to the other end face is 3 µm or more, and more preferably both are 3 µm or more.

The above-described bends of the particles are formed as a result of the crystal growth in (0,0,1) plane being restricted by the presence of impurities as described later, and their bend angles are determined theoretically. However, since the bend angles vary depending on various industrial factors, the bend angles here range from 110° to 160°.

### [Full Width at Half Maximum of Reflectance Peak]

In the hexagonal boron nitride powder, a full width at half maximum of a reflectance peak measured with a variable angle photometer at an angle of incident light of 45° is preferably 80° or less, and more preferably 60° or less. By setting the full width at half maximum to 80° or less, it is possible to increase the glitter of the hexagonal boron nitride powder. The reason is considered to be that the reflection of light is felt more strongly when the light is reflected (scattered) at angles within a narrower range than when reflected at angles over a wider range. The lower limit of the full width at half maximum is not particularly limited, yet may normally be 10° or more. The full width at half maximum can be measured using a variable angle photometer in the manner described in the EXAMPLES section below.

### [Mean Particle Size]

Preferably, the hexagonal boron nitride powder has an mean particle size of 6 µm to 100 µm. When the mean particle size is less than 6 µm, the hexagonal boron nitride powder forms an extremely dense layer when applied to the skin, which ends up deteriorating the glitter property of the glitter pigment such as glass flakes used in combination. In addition, increasing the mean particle size also contributes to increasing the above-described bent particle ratio. Thus, it is preferable to set the mean particle size to 6 µm or more. Further, by setting the mean particle size to 15 µm or more, the reflection of light per particle becomes more pronounced, resulting in a further improvement in glitter property. Thus, it is more preferable to set the mean particle size to 15 µm or more. On the other hand, when the mean particle size is 100 µm or less, adhesion to the skin (skin adhesion) is further improved. Thus, the mean particle size is preferably set to 100 µm or less, and more preferably to 50 µm or less. As used herein, the mean particle size refers to an mean particle size of primary particles, which can be measured according to the method described in the EXAMPLES section below. The mean particle size is controllable via the secondary heating conditions (such as the heating temperature and the processing time).

### [Coarse Particle Ratio]

In the hexagonal boron nitride powder disclosed herein, the proportion of particles having a particle size of 200 µm or more (hereinafter referred to as a "coarse particle ratio") is 0.5 mass% or less. A coarse particle having a particle size of 200 µm or more is an agglomerate of multiple particles adhering to each other, and setting the proportion of such coarse particles to 0.5 mass% or less can further reduce the roughness felt on the skin. The coarse particle ratio is controllable through the choice of crushing means or classifying means.

### [Apparent Thickness]

In the hexagonal boron nitride powder disclosed herein, the apparent thickness of particles constituting the hexagonal boron nitride powder is set in a range of 0.5 µm to 3.0 µm. By setting the apparent thickness to 0.5 µm or more, the glitter property is further improved. On the other hand, by setting the apparent thickness to 3.0 µm or less, the roughness felt on the skin can be further reduced. The apparent thickness is controllable by adjusting the heat treatment conditions during production. As used herein, the apparent thickness refers to an average value of the apparent thicknesses of the particles observed under a microscopic field of view. The apparent thickness can be measured according to the method described in the EXAMPLES section below.

### [Average Aspect Ratio]

In order to further improve the smoothness of the hexagonal boron nitride powder, an average aspect ratio of the hexagonal boron nitride powder (i.e., the ratio of the major axis length to the thickness of the particles) is preferably set in a range of 5 to 30. As used herein, the aspect ratio refers to the result of averaging the aspect ratios of the particles that make up the hexagonal boron nitride powder obtained from the observation of the particles with an electron microscope. In calculating the aspect ratio of each particle, an apparent major axis length and an apparent thickness of the particle in a microscopic field of view can be used as its major axis length and thickness.

### [Production Method]

The production method for the hexagonal boron nitride powder disclosed herein is not particularly limited, yet the following operations (1) to (6) may be sequentially performed. Each operation will be described in detail below.
(1) Mixing
(2) First heating
(3) Cooling
(4) Second heating
(5) Pulverization
(6) Water washing and drying

### (1) Mixing

First, raw material and additive(s) used in the production of a hexagonal boron nitride powder are mixed. As the raw material, a boron compound is used as a boron source and a nitrogen compound as a nitrogen source. As the boron compound, one or both of boric acid and a boron oxide (B₂O₃) are used. The boron compound may further contain a boron carbide. As the nitrogen compound, one or both of urea and urea compound(s) are used. As the urea compound(s), for example, one or both of dicyandiamide and melamine can be used. As the additive(s), at least one selected from the group consisting of Na₂CO₃, K₂CO, MgCO₃, CaCO₃, BaCO₃, MgO, CaO, and BaO is used. Presumably, the use of the additive(s) restricts the crystal growth in (0,0,1) plane, resulting in the formation of boron nitride particles having a bent structure.

### (2) First heating

Then, the mixture of the raw material and additive(s) are heated to a heating temperature of 600 °C to 1200 °C, and held at the heating temperature for 1 hour or more ("first heating"). The first heating causes the raw material to turn into a boron nitride having a turbostratic structure (t-BN). When the heating temperature is below 600 °C, the boron compound and the nitrogen compound as the raw material react with each other, and the reaction does not proceed sufficiently to obtain a boron nitride having a turbostratic structure, resulting in a decrease in the yield of a hexagonal boron nitride in the subsequent second heating. In addition, as described later, when the heating temperature during the first heating is lower than 600 °C, it is impossible to obtain a hexagonal boron nitride powder having a bent particle ratio satisfying the conditions of the present disclosure. On the other hand, if the heating temperature is higher than 1200 °C, the production cost increases, which is economically unsuitable. The first heating may be performed in an inert gas atmosphere, possibly in a nitrogen gas atmosphere.

As used herein, the "turbostratic structure" refers to a state in which it is not fully crystallized. In the X-ray diffraction pattern of a boron nitride having a turbostratic structure, obtained by X-ray diffraction, sharp hexagonal boron nitride peaks do not appear, instead broad peaks appear, indicating that it is not fully crystallized.

### (3) Cooling

After the first heating, the resulting boron nitride powder having a turbostratic structure is cooled once. The method of cooling is not particularly limited, yet may normally be air cooling. Further, in the cooling, it is preferable to cool the boron nitride powder having a turbostratic structure to room temperature.

### (4) Second heating

Then, the cooled boron nitride powder is heated, in the inert gas atmosphere, to a heating temperature of 1500 °C to 2300 °C ("second heating temperature"), and held for 2 hours or more in the heating temperature ("second heating"). The second heating advances the crystallization of the boron nitride and causes the boron nitride having a turbostratic structure (t-BN) to turn into a hexagonal boron nitride (h-BN).

In this case, it is important that the second heating is performed at an average heating rate of 20 °C/min or lower. When the heating rate is 20 °C/min or lower, the occurrence of bending and the crystal growth caused by the presence of additive(s) added to the raw material are promoted, making it possible to obtain a hexagonal boron nitride powder satisfying the conditions for the bent particle ratio specified in the present disclosure. The average heating rate in the second heating is preferably set to 10 °C/min or lower.

In order to produce the boron nitride powder disclosed herein, it is extremely important to perform the first heating in a state in which the raw material is mixed with the additive(s). The reason is considered, for example, as follows.

As described above, through the first heating, the boron compound and the nitrogen compound as the raw material are caused to react to produce a boron nitride having a turbostratic structure. At this point, when the additive(s) has/have been added to the raw material, "boric acid ammonium or the like containing additive components (e.g., Ca)" is generated in addition to the boron nitride having a turbostratic structure. In the presence of the "boric acid ammonium or the like containing additive components", the occurrence of bending and the crystal growth are promoted during the second heating, resulting in an increase in the bent particle ratio. In addition, by adding the additive(s) beforehand prior to the first heating, melt mixing of the raw material and the additive(s) proceeds, and the occurrence of bending and the crystal growth during the second heating is promoted accordingly.

Therefore, in order to ultimately obtain a hexagonal boron nitride powder with a bent particle ratio of 30 % or more, it is necessary to add the additive(s) prior to the first heating. For example, when the additive(s) are added after the first heating and the second heating is performed afterwards, the change from the boron nitride having a turbostratic structure to a hexagonal boron nitride progresses in a state in which the "boric acid ammonium or the like containing additive components" is not present, making it impossible to obtain a bent particle ratio of 30 % or more.

Further, when the heating temperature in the first heating is lower than 600 °C, a boron nitride having a turbostratic structure cannot be formed sufficiently during the first heating. As a result, in the second heating, the reaction does not proceed sufficiently to obtain a hexagonal boron nitride through the crystallization of the boron nitride having a turbostratic structure, making it impossible to obtain a hexagonal boron nitride powder having a bent particle ratio of 30 % or more.

### (5) Pulverization

The hexagonal boron nitride obtained after the second heating has a lumpy bulk body through the heating at high temperature. Thus, the bulk body is pulverized. The pulverization method is not particularly limited, and may follow a conventional method.

### (6) Water washing and drying

After the pulverization, the hexagonal boron nitride is water-washed, sieved, and dried.

### EXAMPLES

The advantageous effects of the present disclosure will be described in detail below based on examples, although the present disclosure is not limited to these examples.

### (Example 1)

Hexagonal boron nitride powder samples were produced according to the following procedure, and their characteristics were evaluated.

First, as the raw material, 100 parts by mass of boric acid, 80 parts by mass of melamine, and 5 parts by mass of boron carbide were mixed with 5 parts by mass of an additive in Table 1.

Then, each mixture of the raw material and the additive was heated in a nitrogen atmosphere to a heating temperature listed in Table 1 and held at the heating temperature for 3 hours to obtain a boron nitride having a turbostratic structure ("first heating"). After the first heating, each product was cooled to room temperature.

Then, each obtained boron nitride having a turbostratic structure was heated in the nitrogen atmosphere to a heating temperature listed in Table 1 and held for 5 hours in the heating temperature ("second heating"). Then, each product was cooled to room temperature to obtain a hexagonal boron nitride. The average heating rate in the second heating was set as presented in Table 1. Each obtained hexagonal boron nitride was pulverized, and water-washed, wet-sieved, dehydrated, and dried in a conventional manner. In the wet sieving, a sieve having an opening of 200 µm was used, and particles that did not pass through the sieve were excluded from the evaluation.

### (Evaluation Method)

For each obtained hexagonal boron nitride powder sample, the bent particle ratio, the mean particle size, the apparent thickness, the coarse particle ratio, and the full width at half maximum of a reflectance peak were measured in the manner described below. In addition, sensory tests were conducted as explained below to evaluate the glitter property, the roughness, and the skin adhesion of each hexagonal boron nitride powder sample, and the glitter property thereof when used in combination with a glitter pigment. The evaluation results are listed in Table 1.

### [Bent Particle Ratio]

The hexagonal boron nitride powder samples were observed with an electron microscope, and for each sample, the number of particles with a bent structure out of 50 randomly selected primary particles were counted. When bending occurs in the primary particles of the hexagonal boron nitride, the hexagonal boron nitride powder sample observed as "with bending" under the electron microscope has a bend angle of 110° to 160° with respect to (0,0,1) crystal plane of the primary particles, virtually without exception. Accordingly, under a microscopic field of view, particles that are observed to be bent when viewed from the side of the particle, and particles that are observed to have a bend line when viewed from the top of the particle, can all be considered as particles having a bent structure at an angle of 110° to 160°. Thus, the bent particle ratio can be determined by (the number of particles having a bent structure/the number of primary particles observed) × 100 (%). In this case, the observation was conducted on a total of 50 particles at x2000 magnification in at least 10 fields of view.

### [Mean Particle Size]

The hexagonal boron nitride powder samples were dispersed in water, and for each sample, the particle size distribution was measured using a laser diffraction particle size analyzer (Mastersizer 3000 available from Spectris). The analytical parameters used were: (i) measurement object: non-spherical, (ii) refractive index: 1.74, (iii) absorptance: 0, (iv) density: 1 g/cm³, and (v) dispersion medium: ethanol (refractive index 1.33). As the mean particle size, 50 % cumulative diameter from the obtained particle size distribution (median size, D50) was used.

### [Apparent Thickness]

The hexagonal boron nitride powder samples were observed with the electron microscope to measure the apparent thickness of primary particles. The observation was conducted at x10000 magnification in 5 fields of view, and the result of averaging the thicknesses of the primary particles observed was used as the apparent thickness.

### [Coarse Particle Ratio]

The "coarse particle ratio" defined as the proportion of particles having a particle size of 200 µm or more was measured as follows. First, the total weight of each hexagonal boron nitride powder sample was measured. Next, all of the hexagonal boron nitride powder samples were dispersed in ethanol and sonicated for 10 minutes to obtain a dispersion. Then, the dispersion was filtered by suction using a sieve having an opening of 200 µm, and then the sieve was dried at 120 °C for 10 minutes and cooled in a desiccator. The coarse particle ratio was determined from the on-sieve weight after the cooling and the total weight of the hexagonal boron nitride powder sample initially measured. That is, the coarse particle ratio is calculated by (on-sieve weight/total weight) × 100 (%).

### [Full Width at Half Maximum of Reflectance Peak]

The boron nitride powder samples were applied to artificial leather, and for each sample, the intensity of reflected light at angles of -90° to 90° for incident light at 45° was measured using a variable angle photometer (GP-200, horizontal rotation, available from Murakami Color Technology Laboratory). A full width at half maximum of a peak in the graph with the angle of reflected light plotted on the x-axis and the intensity of reflected light on the y-axis was defined as a full width at half maximum of a reflected light peak.

### [Glitter property of Hexagonal Boron Nitride Powder Sample]

In this case, 10 mg of each hexagonal boron nitride powder sample was applied to the backs of the hands of 10 testees and evaluated for glitter property. The presence or absence of glitter was evaluated based on whether glitter was visually observed when the back of the hand was tilted. The ratings were as follows, according to the degree of glitter: +++ for excellent, ++ for very good, + for good, - for deficient, andfor poor. Each tested hexagonal boron nitride powder sample earned a rating determined by the highest number of ratings out of 10 testees' results. However, if two or more ratings were equal in number and maximum, the lowest one was taken as the rating for the tested hexagonal boron nitride powder sample.

### [Roughness]

In this case, 10 mg of each hexagonal boron nitride powder sample was taken on the backs of the hands of 10 testees and evaluated for roughness when applied to the backs of their hands with their fingers. The ratings were as follows: ++ for no roughness felt at all, + for slightly inferior to ++ but acceptable, and - for roughness felt.

### [Skin Adhesion]

In this case, 10 mg of each hexagonal boron nitride powder sample was taken on the backs of the hands of 10 testees and visually evaluated for the amount of adhesion to the backs of their hands when applied with their fingers once. The ratings were as follows: ++ for very good, + for good, - for deficient, and -- for poor. Each tested hexagonal boron nitride powder sample earned a rating determined by the highest number of ratings out of 10 testees' results. However, if two or more ratings were equal in number and maximum, the lowest one was taken as the rating for the tested hexagonal boron nitride powder sample.

### [Glitter property when used in combination with Glitter Pigment]

In order to evaluate the glitter property of each hexagonal boron nitride powder sample when used in combination with a glitter pigment, sensory tests of glitter property were conducted under the conditions similar to those of actual cosmetics. Specifically, 20 mass% of each hexagonal boron nitride powder sample, 60 mass% of talc, and 20 mass% of a glitter pigment (glass flakes) were mixed in a mortar to obtain a test composition. Then, 10 mg of each test composition was applied to the backs of the hands of 10 testees to determine the presence or absence of glitter. The presence or absence of glitter was evaluated based on whether glitter was visually observed when the back of the hand was tilted. The ratings were as follows, according to the degree of glitter as in the case of testing each hexagonal boron nitride powder sample alone: +++ for excellent, ++ for very good, + for good, - for deficient, and -- for poor. Each tested hexagonal boron nitride powder sample earned a rating determined by the highest number of ratings out of 10 testees' results. However, if two or more ratings were equal in number and maximum, the lowest one was taken as the rating for the tested hexagonal boron nitride powder sample. Although the above examples have been described in the context of each hexagonal boron nitride powder sample having a mix proportion of 20 mass%, the present inventors also confirmed that the same effect as presented in Table 1 was obtained for ordinary mix proportions of 3 mass% to 30 mass%.

**Table 1**

| No. | Production conditions | | | | Evaluation results | | | | | \| Results of sensory tests | | | | Remarks |
|---|---|---|---|---|---|---|---|---|---|---|---|---|---|---|
| | | | | | Hexagonal boron nitride powder sample | | | | | | | | Combined use with glitter pigment | |
| | Additive | First heating | Second heating | | Bent particle ratio* (%) | Mean particle size (µm) | Apparent thickness (µm) | Coarse particle ratio (mass%) | Full width at half maximum (°) | Glitter property | Roughness | Skin adhesion | Pearlescence | |
| | | Heating temp. (°C) | Heating temp. (°C) | Average heating rate (°C/min) | | | | | | | | | | |
| 1 | CaCO₃ | 850 | 2000 | 5 | 78 | 23 | 0.8 | 0.2 | 50 | ++ | ++ | ++ | ++ | Example |
| 2 | CaCO₃ | 850 | 2000 | 5 | 48 | 30 | 0.8 | 0.2 | 50 | ++ | ++ | ++ | ++ | Example |
| 3 | CaO | 700 | 2000 | 10 | 81 | 30 | 0.8 | 0.2 | 63 | ++ | ++ | ++ | ++ | Example |
| 4 | CaCO₃ | 850 | 1900 | 10 | 83 | 6 | 0.8 | 0.2 | 75 | + | ++ | ++ | + | Example |
| 5 | CaCO₃ | 850 | 2000 | 10 | 79 | 70 | 0.8 | 0.2 | 60 | ++ | ++ | + | ++ | Example |
| 6 | none | 850 | 2000 | 10 | 18 | 9 | 0.5 | 0 | 99 | -- | ++ | ++ | - | Comparative example |
| 7 | CaCO₃ | 850 | 2050 | 20 | 30 | 12 | 0.4 | 0.3 | 80 | + | ++ | ++ | + | Example |
| 8 | CaCO3 | 850 | 2000 | 25 | 21 | 15 | 0.8 | 0.2 | 90 | -- | ++ | ++ | -- | Comparative example |
| 9 | CaCO₃ | 850 | 2000 | 18 | 52 | 20 | 0.8 | 0.2 | 78 | + | ++ | ++ | + | Example |
| 10 | CaCO₃ | 850 | 2050 | 100 | 16 | 11 | 0.6 | 0.1 | 88 | -- | ++ | ++ | -- | Comparative example |
| 11 | BaCO₃ | 850 | 2000 | 3 | 62 | 16 | 0.5 | 0.2 | 55 | ++ | ++ | ++ | ++ | Example |
| 12 | MgCO₃ | 700 | 2000 | 10 | 69 | 22 | 0.5 | 0.4 | 78 | ++ | ++ | ++ | ++ | Example |
| 13 | CaCO₃ | 850 | 2000 | 10 | 5 | 4 | 0.8 | 0.2 | 120 | -- | ++ | ++ | -- | Comparative example |
| 14 | CaCO₃ | 850 | 2000 | 5 | 75 | 130 | 1 | 0.5 | 50 | ++ | ++ | + | ++ | Example |
| 15 | CaCO₃ | 850 | 2000 | 15 | 60 | 90 | 0.8 | 0.8 | 52 | ++ | + | ++ | ++ | Example |

| | | | | | | | | | | | | | | |
|---|---|---|---|---|---|---|---|---|---|---|---|---|---|---|
| * Bent particle ratio at 110° to 160°. | | | | | | | | | | | | | | |

Please note, powder No. 15 falls outside the scope of claim 1, due to the proportion of particles having a particle size of 200 µm or more being greater than 0.5 mass%, but is retained for comparative purposes. As can be seen from the results listed in Table 1, each of hexagonal boron nitride powder samples satisfying the conditions of the present disclosure exhibited excellent glitter property. In contrast, the hexagonal boron nitride powder samples Nos. 6, 8, 10, and 13 which did not meet the conditions of the present disclosure had inferior glitter property.

For powder No. 6, since any of the predetermined additives was not used in production, the bent particle ratio was low, resulting in inferior glitter property. For powder Nos. 8 and 10, since the heating rate in the second heating was higher than 20 °C/min, primary particles grown fast and the facet growth did not proceed sufficiently, resulting in a low bent particle ratio. The mean particle size was less than 15 µm because the second heating was performed at a high temperature (2050 °C), and consequently the volatilization rate of boron oxide, which is responsible for particle growth, was greater than the particle growth rate. For powder No. 13, since pulverization was performed excessively until the mean particle size was 4 µm, the bent ratio was as low as 5 %.

In addition, among the hexagonal boron nitride powder samples in our examples, those powder samples having an mean particle size of 6 µm to 100 µm were superior in skin adhesion to powder No. 14 having an mean particle size of 130 µm. Moreover, among the hexagonal boron nitride powder samples in our examples, those powder samples having a coarse particle ratio of 0.5 mass% or less had less roughness than powder No. 15 having a coarse particle ratio of 0.8 mass%.

Experiments were also conducted using a lame agent instead of glass flakes as a glitter raw material, yet the results were similar to those using glass flakes.

### (Example 2)

Hexagonal boron nitride powder samples were produced under the production conditions listed in Table 2. The conditions were otherwise the same as in Example 1. Then, each obtained hexagonal boron nitride powder sample was evaluated in the same way as in Example 1. However, as the bent particle ratio, the number ratio of particles having a bent structure at an angle of 110° to 130° with respect to (0,0,1) crystal plane of the particles ("second bent particle ratio") was measured. The second bent particle ratio was determined by image analysis (three-dimensional analysis) of images obtained by observing each hexagonal boron nitride powder sample under an electron microscope according to a conventional method.

As can be seen from the results listed in Table 2, those hexagonal boron nitride powder samples having a second bent particle ratio of 60 % or more exhibited much better glitter property than that of other hexagonal boron nitride powder samples having a second bent particle ratio of less than 60 %.

**Table 2**

| No. | Production conditions | | | | Evaluation results | | | | | Results of sensory tests | | | | Remarks |
|---|---|---|---|---|---|---|---|---|---|---|---|---|---|---|
| | | | | | Hexagonal boron nitride powder sample | | | | | | | | Combined use with glitter pigment | |
| | Additive | First heating | Second heating | | Bent particle ratio* (%) | Mean particle size (µm) | Apparent thickness (µm) | Coarse particle ratio (mass%) | Full width at half maximum (°) | Glitter property | Roughness | Skin adhesion | Pearlescence | |
| | | Heating temp. (°C) | Heating temp. (°C) | Average heating rate (°C/min) | | | | | | | | | | |
| 16 | CaCO₃ | 850 | 2000 | 5 | 82 | 23 | 0.8 | 0.2 | 40 | + + + | + + | + + | + + + | Example |
| 17 | CaO | 700 | 2000 | 10 | 80 | 30 | 0.8 | 0.2 | 50 | + + + | + + | + + | + + + | Example |
| 18 | CaCO₃ | 850 | 2000 | 25 | 42 | 20 | 0.8 | 0.2 | 70 | + | + + | + + | + | Example |
| 19 | BaCO₃ | 850 | 2000 | 3 | 61 | 16 | 0.5 | 0.2 | 50 | + + + | + + | + + | + + + | Example |
| 20 | MgCO₃ | 700 | 2000 | 10 | 72 | 22 | 0.5 | 0.4 | 70 | + + + | + + | + + | + + + | Example |

| | | | | | | | | | | | | | | |
|---|---|---|---|---|---|---|---|---|---|---|---|---|---|---|
| * Bent particle ratio at 110° to 130°. | | | | | | | | | | | | | | |

## Claims

1. A hexagonal boron nitride powder comprising hexagonal boron nitride particles, wherein
among the hexagonal boron nitride particles, a number ratio of particles having a bent structure at an angle of 110° to 160° with respect to (0,0,1) crystal plane of the particles is 30 % or more;
the proportion of particles having a particle size of 200 µm or more is 0.5 mass% or less; and
the avearge apparent thickness of the hexagonal boron nitride particles is 0.5 µm to 3.0 µm.

2. The hexagonal boron nitride powder according to claim 1, wherein among the hexagonal boron nitride particles, a number ratio of particles having a bent structure at an angle of 110° to 130° with respect to (0,0,1) crystal plane of the particles is 60 % or more.

3. The hexagonal boron nitride powder according to claim 1 or 2, wherein a full width at half maximum of a reflectance peak of the hexagonal boron nitride powder measured with a variable angle photometer at an angle of incident light of 45° is 80° or less.

4. The hexagonal boron nitride powder according to any one of claims 1 to 3, wherein the hexagonal boron nitride powder has a mean particle size of 6 µm to 100 µm.

5. A cosmetic product comprising the hexagonal boron nitride powder of any one of claims 1 to 4.

6. Use of the hexagonal boron nitride powder according to any one of claims 1 to 4 in cosmetics.

## Patentansprüche

1. Hexagonales Bornitridpulver, hexagonale Bornitridpartikel umfassend, wobei
unter den hexagonalen Bornitridpartikeln ein Zahlenverhältnis von Partikeln mit einer gebogenen Struktur in einem Winkel von 110° bis 160° in Bezug auf die (0,0,1)-Kristallebene der Partikel 30 % oder mehr beträgt;
der Anteil von Partikeln mit einer Partikelgröße von 200 µm oder mehr 0,5 Massen-% oder weniger beträgt; und
die durchschnittliche scheinbare Dicke der hexagonalen Bornitridpartikel 0,5 µm bis 3,0 µm beträgt.

2. Hexagonales Bornitridpulver gemäß Anspruch 1, wobei unter den hexagonalen Bornitridpartikeln ein Zahlenverhältnis von Partikeln mit einer gebogenen Struktur in einem Winkel von 110° bis 130° in Bezug auf die (0,0,1)-Kristallebene der Partikel 60 % oder mehr beträgt.

3. Hexagonales Bornitridpulver gemäß Anspruch 1 oder 2, wobei eine Halbwertsbreite eines Reflexionspeaks des hexagonalen Bornitridpulvers, gemessen mit einem variablen Winkelphotometer bei einem Lichteinfallswinkel von 45°, 80° oder weniger beträgt.

4. Hexagonales Bornitridpulver gemäß einem der Ansprüche 1 bis 3, wobei das hexagonale Bornitridpulver eine mittlere Partikelgröße von 6 µm bis 100 µm aufweist.

5. Kosmetikprodukt, das hexagonale Bornitridpulver nach einem der Ansprüche 1 bis 4 umfassend.

6. Verwendung des hexagonalen Bornitridpulvers gemäß einem der Ansprüche 1 bis 4 in Kosmetika.

## Revendications

1. Poudre de nitrure de bore hexagonal comprenant des particules de nitrure de bore hexagonal,
parmi les particules de nitrure de bore hexagonal, un rapport numérique de particules comportant une structure courbée selon un angle de 110° à 160° par rapport au plan cristallin (0,0,1) des particules étant supérieur ou égal à 30 % ;
ladite proportion de particules comportant une taille de particule supérieure ou égale à 200 µm étant inférieure ou égale à 0,5 % en masse ; et
ladite épaisseur apparente moyenne des particules hexagonales de nitrure de bore hexagonal étant de 0,5 µm à 3,0 µm.

2. Poudre de nitrure de bore hexagonal selon la revendication 1, parmi les particules de nitrure de bore hexagonal, un rapport numérique de particules comportant une structure courbée selon un angle de 110° à 130° par rapport au plan cristallin (0,0,1) des particules étant supérieur ou égal à 60%.

3. Poudre de nitrure de bore hexagonal selon la revendication 1 ou 2, ladite largeur totale à mi-hauteur d'un pic de réflectance de la poudre de nitrure de bore hexagonal mesurée avec un photomètre à angle variable à un angle de lumière incidente de 45° étant inférieure ou égale de 80°.

4. Poudre de nitrure de bore hexagonal selon l'une quelconque des revendications 1 à 3, ladite poudre de nitrure de bore hexagonal comportant une taille de particule moyenne de 6 µm à 100 µm.

5. Produit cosmétique comprenant la poudre de nitrure de bore hexagonal selon l'une quelconque des revendications 1 à 4.

6. Utilisation de la poudre de nitrure de bore hexagonal selon l'une quelconque des revendications 1 à 4 en cosmétique.
